# EUROPEAN PATENT SPECIFICATION

(11) **EP 1 064 254 B1**
(45) Date of publication and mention of the grant of the patent: **17.10.2007**
(21) Application number: 99908728.1
(22) Date of filing: 25.03.1999
(51) Int. Cl.: C07C 229/00

(54) **ALIPHATIC AMINO CARBOXYLIC AND AMINO PHOSPHONIC ACIDS, AMINO NITRILES AND AMINO TETRAZOLES AS CELLULAR RESCUE AGENTS**
ALIPHATISCHE AMINOSÄURE UND AMINOPHOSPHONSÄURE, AMINONITRILE UND AMINOTETRAZOLE ALS ZELLULARES RETTUNGSMITTEL
ACIDES AMINO PHOSPHONIQUES ET AMINO CARBOXYLIQUES ALIPHATIQUES, AMINO NITRILES ET AMINO TETRAZOLES UTILISES EN TANT QU'AGENTS DE RETABLISSEMENT DE FONCTIONS CELLULAIRES

(30) Priority: 26.03.1998 US 79488 P; 26.03.1998 US 79489 P
(43) Date of publication of application: 03.01.2001
(73) Proprietor: University of Saskatchewan, Saskatoon, SK S7N 4J8 (CA)
(72) Inventor: PATERSON, I., Alick DI, deceased (CA); DYCK, Lillian, E., Saskatoon, Saskatchewan S7N 0N8 (CA); DAVIS, Bruce, A., Saskatoon, Saskatchewan S7K 4K9 (CA); LIU, Ya-Dong, Fremont, CA 94536 (US); DURDEN, David, A., Calgary, Alberta T2K 5P3 (CA); BOULTON, Alan, A., Nanoose Bay, British Columbia, V9P 9G2 (CA)
(74) Representative: Albrecht, Thomas
(86) International application number: PCT/CA1999/000250
(87) International publication number: WO 1999/048858

(56) References cited:
- WO-A-92/15551
- WO-A-96/05286
- WO-A-97/45115
- DE-A- 1 567 221
- DE-A- 2 052 256
- DE-A- 2 442 239
- DE-A- 2 555 769
- DE-A- 4 100 856
- DE-B- 1 139 738
- FR-A- 2 451 913
- US-A- 3 991 208
- US-A- 4 548 726
- KUNIKO NISHIMURA ET AL.: "Inactivation of monoamine oxidase B by analogues of the anticonvulsant agent milacemide (2-(n-pentylamino)acetamide)" JOURNAL OF MEDICINAL CHEMISTRY., vol. 36, no. 4, 1993, pages 446-448, XP002109875 WASHINGTON US
- CHEMICAL ABSTRACTS, vol. 55, no. 15, 1961 Columbus, Ohio, US; abstract no. 14441b, GERHARD SATZINGER: "5-Substituted and 1,5-condensed tetrazoles." page 14441; column 1; XP002109056 & ANN., vol. 638, 1960, pages 159-173,
- FREDERICK LEONARD ET AL.: "Potential antiviral agents." JOURNAL OF THE AMERICAN CHEMICAL SOCIETY., vol. 78, no. 6, 20 March 1956 (1956-03-20), pages 1199-1201, XP002109053 DC US
- HANS-G. BOIT: "BEILSTEINS HANDBUCH DER ORGANISCHEN CHEMIE, 4. Edition, 3. suppl., vol. 4, part 2" 1963 , SPRINGER-VERLAG XP002109054 page 1137, paragraph 9
- REINER LUCKENBACH: "BEILSTEINS HANDBUCH DER ORGANISCHEN CHEMIE, 4. Edition, 4. suppl., vol. 4, part 3" 1980 , SPRINGER-VERLAG XP002109055 page 2386, paragraph 3
- SHIGEYOSHI MIYAGISHI ET AL.: "Phase transition of N-acyl amino acids with different acyl group" BULLETIN OF THE CHEMICAL SOCIETY OF JAPAN., vol. 59, no. 2, 1986, pages 557-562, XP002109876 JAPAN PUBLICATIONS TRADING CO. TOKYO., JP ISSN: 0009-2673
- CHEMICAL ABSTRACTS, vol. 92, no. 23, 1980 Columbus, Ohio, US; abstract no. 192462w, KIRINO, OSAMU ET AL.: "Studies on anti-fusarium disease activity of aminonitrile derivatives......" page 167; column 2; XP002122173 & AGRIC. BIOL. CHEM., vol. 44, no. 1, 1980, pages 31-34,
- JOSEPH CORSE ET AL.: "N-substituted 2-methoxy-6-chloro-9-aminoacridines derived from unsymmetrical aliphatic amines" JOURNAL OF THE AMERICAN CHEMICAL SOCIETY., vol. 68, 1946, pages 1905-1910, XP002122172 DC US
- CHEMICAL ABSTRACTS, vol. 74, no. 8, 1971 Columbus, Ohio, US; abstract no. 32910r, ISHIZUKA, TETSUO ET AL.: "Alkyl-beta-amines from long-chain olefins" page 70; column 1; XP002122174 & CHIM. PHYS. APPL. PRAT. AG. SURFACE, C. R. CONGR. INT. DETERG., 5TH, vol. 1, 1968, pages 183-191,
- CHEMICAL ABSTRACTS, vol. 55, no. 8, 1961 Columbus, Ohio, US; abstract no. 7275d, SEIZABURO SAKAKIBARA ET AL.: "Cationic surface-active agents from long-chain alkyl amines and acrylonitrile" page 7275; column 1; XP002122175 & YUKAGAKU, vol. 6, 1957, pages 263-266,
- CHEMICAL ABSTRACTS, vol. 55, no. 15, 24 July 1961 (1961-07-24) Columbus, Ohio, US; abstract no. 14441b, GERHARD SATZINGER: "5-substituted and 1,5-condensed tetrazoles." page 14441; column 1; XP002122176 & ANN., vol. 638, 1960, pages 159-173,
- CHEMICAL ABSTRACTS, vol. 55, no. 23, 13 November 1961 (1961-11-13) Columbus, Ohio, US; abstract no. 23837e, HISASHI TAKAHASHI: "Effects of derivatives of gamma-aminobutyric acid ...." page 23837; column 1; XP002122177 & NIPPON SEIRIGAKU ZASSHI, vol. 23, 1961, pages 417-425,

## Description

The invention relates to novel aliphatic amino carboxylic and amino phosphonic acids, amino nitriles and amino tetrazoles, their salts, compositions containing such compounds and to the use of such compounds as cellular rescue agents in human and veterinary medicine.

### BACKGROUND OF THE INVENTION

Neurodegenerative disorders (for example, chronic disorders such as Alzheimer's disease, Parkinson's disease, Huntington's disease, Picks's disease, amyotrophic lateral sclerosis and glaucoma as well as acute injuries like stroke, head trauma, Bell's palsy and spinal cord injuries) are now believed to involve apoptotic processes.

Deprenyl, the aliphatic propargylamines, their respective desmethyl analogues and rasagiline have been shown to protect and rescue damaged neurons in several models of degeneration [1-16]. The propargyl group was thought to be a requirement for the protective or rescuing activity of these drugs. Previous studies have examined the N-demethylation and/or depropargylation of these drugs [7, 17].

It has been known for some time that some aliphatic and aromatic acetylenic compounds react with P450 enzymes. One of these reactions results in oxidation of the terminal carbon of the acetylenic functional group to form the corresponding acid [18-20]. The possibility of oxidation of the N-acetylene group of the aliphatic propargylamines to form carboxylic acid metabolites has not been previously addressed. The potential of related acidic compounds (the amino phosphonic acids and amino tetrazoles) and precursors to such compounds (amino nitriles and amino esters) as antiapoptotic agents had also not been previously considered.

The present inventors have found that aliphatic amino carboxylic and amino phosphonic acids, amino nitriles and amino tetrazoles are antiapoptotic agents and may be useful as cellular rescue agents in human and animal treatments.

### SUMMARY OF THE INVENTION

The present invention provides a compound of the general formula (I), 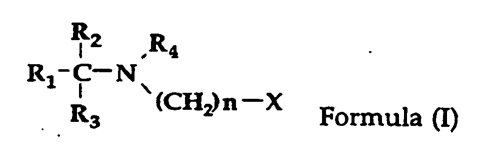 wherein:
R₁ is (CH₂)ₘCH₃ where m is 0 or an integer in the range from 1 to 16,
R₂ = CH₃,
R₃=H,
R₄ = H or CH₃,
n is 2,
X is carboxyl (COOH) or carbalkoxy (COOR₅), cyano (CN), phosphonic acid (PO₃H₂), phosphonate ester (PO₃[R₅]₂) or 5-tetrazole,
R₅ is lower alkyl having from 1 to 5 carbon atoms, and
R₁, R₂ and R₃ differ from one another so that the carbon atom to which they are attached is chiral and the compound of Formula I is a substantially pure enantiomer in the R configuration when X is COOH, COOR₅, CN or tetrazole, or a substantially pure enantiomer in the S configuration when X is PO₃H₂ or P0₃[R₅]₂,
or a pharmaceutically acceptable salt thereof.

Furthermore, the invention provides 3-(2-propylmethylamino)propionic acid; methyl 3-(1-hexylmethylamino)propionate; 3-(3-pentylamino)propionitrile; 3-(3-pentylmethylamino)propionitrile; 2-(2-propylamino)ethanephosphonic acid; and 2-(2-propylmethylamino)ethanephosphonic acid.

Moreover, the invention provides the use of a compound selected from: 3-(2-propylamino)propionic acid; 3-(1-hexylamino)propionic acid; 3-(2-propylmethylamino)propionic: acid; 3-(1-hexylmethylamino)propionic acid; 2-(2-propylamino)ethanephosphonic acid; and 2-(2-propylmethylamino)ethanephosphonic acid for the manufacture of a medicament for the treatment or prevention of a disease in which cell death occurs by apoptosis.

Compounds of the general formula I in which R₁, R₂ and R₃ differ from one another are chiral. It has been found that the R-enantiomers of some of these classes or sub-classes of compounds (and the S-enantiomers for other classes or subclasses) are useful as cellular rescue agents for the treatment and prevention of diseases in which cell death occurs by apoptosis, such as in many neurodegenerative disorders. For a particular class or subclass of compounds the inactive enantiomer does not prevent apoptosis but can antagonize the antiapoptotic actions of the active enantiomers, and are useful as research tools. The achiral compounds also display cellular rescue properties.

The present invention also relates to the use of compounds of general formula I, as defined above, and salts thereof, as cellular rescue agents for the manufacture of a medicament for the treatment and prevention of diseases in which cell death occurs by apoptosis including but not limited to, stroke, head trauma, Bell's palsy, spinal cord and other nerve crush injuries, Alzheimer's disease, Parkinson's disease, Pick's disease, amyotrophic lateral sclerosis, Huntington's disease, multiple sclerosis, cardiac myopathies, nephropathy, retinopathy, diabetic complications, glaucoma, as well as idiopathic neuropathies.

### DETAILED DESCRIPTION OF PREFERRED EMBODIMENTS

### Compounds of the Invention

The present invention provides compounds of the general formula I, 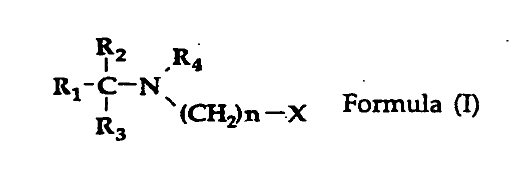 wherein:
R₁ is (CH₂)ₘCH₃ where m is 0 or an integer in the range from 1 to 16,
R₂ = CH₃,
R₃=H,
R₄=H or CH₃,
n is 2,
X is carboxyl (COOH) or carbalkoxy (COOR₅), cyano (CN), phosphonic acid (PO₃H₂), phosphonate ester (PO₃[R₅]₂) or 5-tetrazole,
R₅ is lower alkyl having from 1 to 5 carbon atoms, and
R₁, R₂ and R₃ differ from one another so that the carbon atom to which they are attached is chiral and the compound of Formula I is a substantially pure enantiomer in the R configuration when X is COOH, COOR₅, CN or tetrazole, or a substantially pure enantiomer in the S configuration when X is PO₃H₂ or PO₃[R₅]₂.
or a pharmaceutically acceptable salt thereof.

Preferred compounds of the invention include:
(R)-3-(2-Heptylamino)propionic acid;
(R)-3-(2-Heptylmethylamino)propionic acid;
Methyl (R)-3-(2-heptylamino)propionate;
Methyl (R)-3-(2-heptyhnethylamino)propionate;
(R)-3-(2-Heptylamino)propionitrile;
(R)-3-(2-Heptylmethylamino)propionitrile;
(R)-2-(2-Heptylamino)ethane-5-tetrazole,
(S)-2-(2-Heptylamino)ethanephosphonic acid;
(S)-2-(2-Heptylmethylamino)ethanephosphonic acid
3-(2-Propylmethylamino)propionic acid;
Methyl 3-(1-hexylmethylamino)propionate;
3-(3-Pentylamino)propionitrile;
3-(3-Pentylmethylamino)propionitrile;
2-(2-Propylamino)ethanephosphonic acid; an
2-(2-Propylmethylamino)ethanephosphonic acid.

Methods of resolving the racemates are known. Suitable methods include fractional crystallization, derivatization of the racemate followed by stereospecific enzymatic removal of the attached group, and chromatography. It is preferred, however, to make chiral compounds of formula I from chiral reactants, using reactions that do not destroy the stereochemistry. When referring to enantiomers, it is preferred that an enantiomer shall not contain more than 3% of the compound of the opposite configuration. It is particularly preferred that an enantiomer contain less than 1% of the corresponding enantiomer of the opposite configuration.

Some of the inactive enantiomers for a given class or subclass of compounds strongly antagonize the antiapoptotic actions of the active enantiomers, and are useful as research tools.

The invention extends to salts of compounds of formula I. For administration the salts should be pharmaceutically acceptable, but other salts may be useful, for example, in synthesis or for purification.

### Methods of Preparing Compounds of the Invention

Compounds of the invention can be prepared in a variety of different ways. One process involves the addition of a primary amine of formula (II) 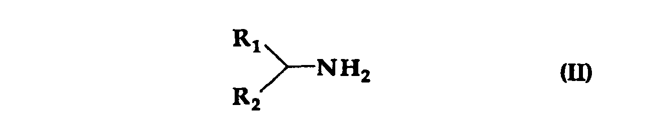 across the olefinic double bond of α,β-unsaturated carboxylic acid esters (such as methyl acrylate), of vinylphosphonic acids esters or of α,β-unsaturated nitriles (such as acrylonitrile) of formula (III)[21],

H₂C₌CH- X (III)

wherein X is a polarized group such as carboxylic ester, phosphonic ester or nitrile to give the corresponding N-alkylamino propionic esters, ethanephosphonic esters and propionitriles.

It is possible to use an amine of the formula (II) in which R₁ differs from R₂ in the form of a racemate and to separate the enantiomers subsequently, but it is preferred to use an amine in substantially enantiomerically pure form.

Chiral primary amines (R- or S- forms) were prepared by recrystallization of the tartrates of the racemates from methanol [22], except for (R) and (S)-2-butylamines which were purchased from Aldrich Chemical Co. Enantiomeric purity was determined using gas chromatography with a chiral capillary column and chiral derivatizing agent [23]. In analogy to the above, chiral primary amines can also be added to the C-C double bond of methyl acrylate, diethyl vinylphosphonate or acrylonitrile to give the corresponding chiral methyl N-alkylaminopropionate, chiral diethyl N-alkylaminoethanephosphonate or chiral N-alkylaminopropionitrile.

In one embodiment, an excess of a chiral amine adds to the olefinic bond of acrylonitrile, as depicted in the following scheme.

### Excess Amine and Acrylonitrile:

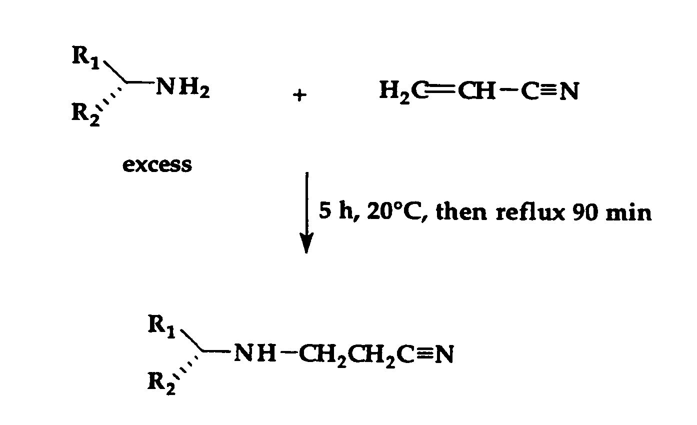

where R₁ = hydrogen, methyl or ethyl and
R₂ = methyl, ethyl, propyl, butyl, pentyl, hexyl.

In another embodiment an excess of the amine can be added to the olefinic double bond of diethyl vinylphosphonate to give the corresponding diethyl N-alkylaminoethanephosphonate. Hydrochloric acid hydrolysis of the aminophosphonic diester yields the corresponding aminophosphonic acid as the hydrochloride salt.

In yet another embodiment an excess of the amine can be added to the olefinic double bond of methyl acrylate to give the corresponding methyl N-alkylaminopropionate [24]. Hydrolysis of the carboxylate ester with hydrochloric acid produces an amino acid as its hydrochloride salt, in accordance with the following reaction scheme:

### Hydrolysis of Esters:

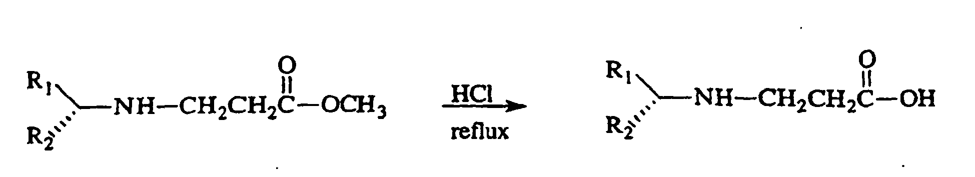

A second process to give compounds which contain only one carbon atom between the nitrogen atom and the functional group involves condensing a primary aliphatic amine of formula (II) with a bromomethyl reactant of formula (IV)

LCH₂X (IV)

wherein L is a leaving group, for example a halide, tosyl or mesyl group (bromide is preferred), and X is carboalkoxy (carbomethoxy or carbethoxy is preferred), nitrile or phosphodiethoxy. Again, the amine can be used in racemic or enantiomerically pure form. In one preferred embodiment, two equivalents of the amine are reacted with one equivalent of the bromomethyl analogue of formula (IV) to form the required aminomethanecarboxylate (glycine) ester and the hydrobromide salt of the amine, which can be isolated and reused, in accordance with the following reaction scheme.

Two Equivalents of Amine and One Equivalent of Bromomethyl Ester in Ether:

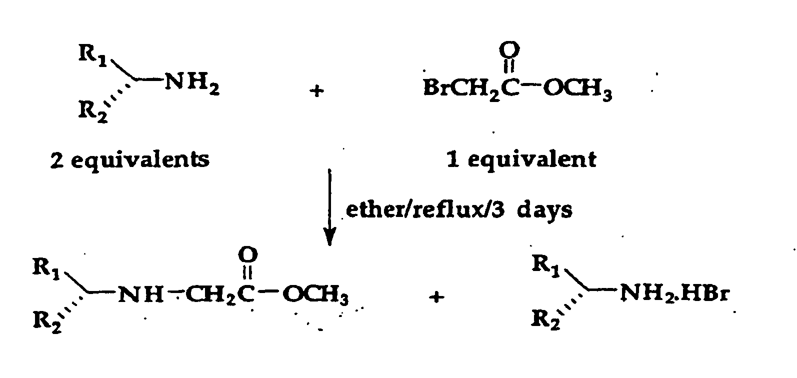

In another preferred embodiment two equivalents of the amine are reacted with one equivalent of the bromomethyl analogue of formula (IV) to form the required aminomethane nitrile (aminoacetonitrile) and the hydrobromide salt of the amine, which can also be isolated and reused, in accordance with the following reaction scheme.

### Two Equivalents of Amine and One Equivalent of Bromoacetonitrile in Ether:

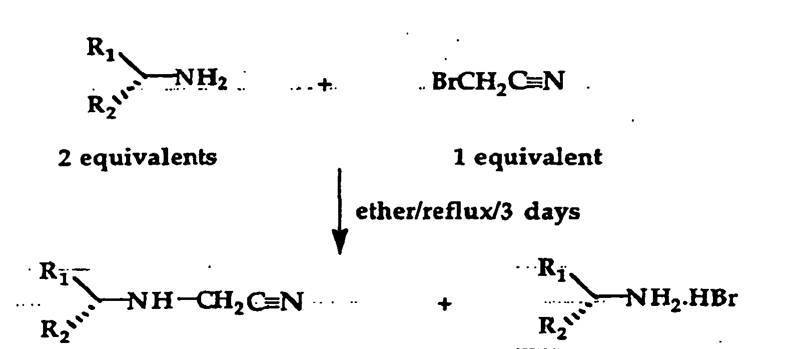

Another route to compounds of the invention involves trifluoroacetylation of the amine, followed by reaction with bromoethyl analogues of esters of carboxylic acids or nitriles of formula (V).

L(CH₂)nX (V)

wherein L is a leaving group, for example a halide, tosyl or mesyl group (bromide is preferred), n is 2 and X is carboalkoxy (methoxy or ethoxy is preferred), nitrile or phosphodiethoxy.

The amine can be used in racemic or enantiomerically pure form. The amine is reacted with trifluoroacetic anhydride or a trifluoroacetyl halide in an inert organic solvent, for instance a chlorinated hydrocarbon such as methylene dichloride, chloroform or carbon tetrachloride, and a base, for example an organic base such as triethylamine. The N-trifluoroacetylamine is then refluxed with a bromo compound of formula (V), suitably in the presence of a base such as potassium t-butoxide in a polar solvent, for example acetonitrile, and in the presence of a crown ether catalyst, for example 18-crown-6. The product of this reaction is then hydrolyzed, suitably by reaction with a base such as an alkali metal hydroxide in an alcoholic solution.

Tetrazole compounds of the invention were prepared by the addition of azide ion to a nitrile triple bond [25]. Again, the amine can be used in racemic or enantiomerically pure form. In one preferred embodiment azide ion is generated by the slow addition of aluminum chloride to a solution/suspension of sodium azide in tetrahydrofuran at 0°C. To the resulting solution of aluminum azide is added the nitrile (prepared as in the pathways described above) in tetrahydrofuran at room temperature, followed by stirring for 2 hours and then gentle refluxing for several hours, according to the following scheme.

### Addition of Azide Ion to Nitrile:

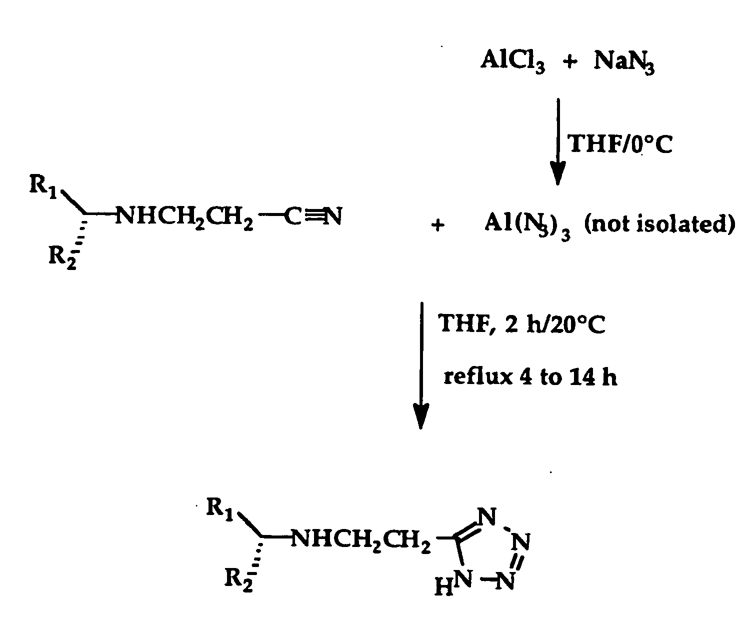

N-Methylation of the various secondary amines described above is achieved by reductive methylation using formaldehyde and sodium phosphite [26]. Again, the amine can be used in racemic or enantiomerically pure form. An amino carboxylate ester or amino nitrile (as the hydrochloride salt or free base) is dissolved in aqueous sodium dihydrogen phosphite and reacted with an excess of 37% aqueous formaldehyde at 60°C for 15 min. The product is isolated by ether extraction after basification of the ice-cold reaction mixture with sodium hydroxide. A preferred embodiment is shown in the following reaction scheme.

### N-Methylation of an Amino Ester:

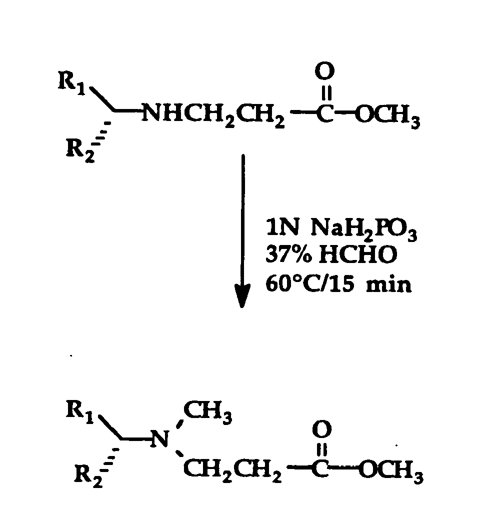

Yet another preferred embodiment is shown in the following reaction scheme.

### N-Methylation of an Amino Nitrile:

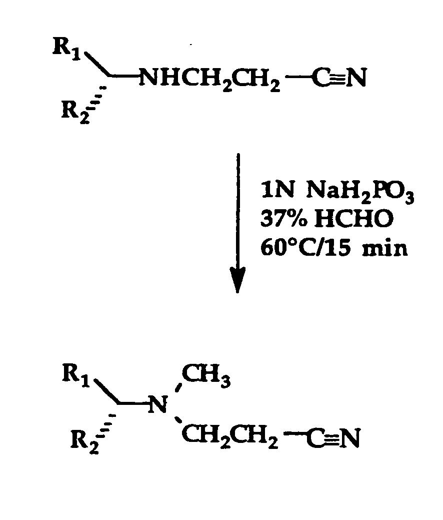

### Therapeutic Methods of the Invention

As hereinbefore mentioned, the compounds of the formula I (as described above) have many therapeutic applications.

The present invention provides a use of a compound as defined above to prepare a medicament to treat or prevent a condition wherein cell death occurs by apoptosis.

Conditions wherein cell death occurs by apoptosis includes, but are not limited to, stroke, head trauma, Bell's palsy, spinal cord and other nerve crush injuries, Alzheimer's disease, Parkinson's disease, Pick's disease, amyotrophic lateral sclerosis, Huntington's disease, multiple sclerosis, cardiac myopathies, nephropathy, retinopathy, diabetic complications, glaucoma, as well as idiopathic neuropathies.

### Pharmaceutical Compositions

The compounds defined above may be formulated into pharmaceutical compositions for administration to subjects in a biologically compatible form suitable for administration *in vivo.* The compositions containing the compounds of the invention can be prepared by per se known methods for the preparation of pharmaceutically acceptable compositions which can be administered to subjects, such that an effective quantity of the active substance is combined in a mixture with a pharmaceutically acceptable vehicle. Suitable vehicles are described, for example, in Remington's Pharmaceutical Sciences (Remington's Pharmaceutical Sciences, Mack Publishing Company, Easton, Pa., USA 1985). On this basis, the compositions include, albeit not exclusively, solutions of the substances in association with one or more pharmaceutically acceptable vehicles or diluents, and contained in buffered solutions with a suitable pH and iso-osmotic with the physiological fluids.

The active substance may be administered in a convenient manner such as by injection (subcutaneous, intravenous, etc.), oral administration, inhalation, transdermal application, or rectal administration.

In oral administration, the compounds may be administered as tablets, coated tablets, gelatine capsules, capsules, cachets, and solutions or suspensions to be taken orally. The compounds can also be administered parenterally or through any other suitable administrative route such as intravenous, subcutaneous, depot injections; intramuscular; intrathecal, intraventricular, intra-articular, rectal (suppository, enema), sublingual, buccal, intra-ocular, intra-vitreo, transdermal (skin patch), nasal drops (nebulizer, insufflation), liposomal delivery systems. The daily dosage could likely range from 1 to 100 mg.

Accordingly, in another aspect, the present invention provides a pharmaceutical composition comprising a compound as defined above in admixture with a suitable diluent or carrier. The compound may be achiral or a substantially enantiomerically pure R- or S-enantiomer, or a pharmaceutically acceptable salt thereof, in admixture with pharmaceutically acceptable diluents or carriers. The compositions are useful in the treatment or prevention of conditions in which cell death occurs by apoptosis.

In another aspect, the present invention provides a commercial package containing as active ingredient a compound as defined above or a pharmaceutically acceptable salt thereof, together with instructions for its use for the treatment or prevention of a condition in which cell death occurs by apoptosis.

### EXAMPLES

### Example 1:

### In Vitro Protocol for Assessing the Antiapontotic Capacity of Various Compounds in Cerebellar Granule Cells:

The following biological data demonstrate that the compounds of the invention exhibit antiapoptotic properties. Data for compounds not falling within the claims are given for comparison only.

Cultures of cerebellar granule cells (CGC) can be induced into apoptosis by the addition of a high concentration of cytosine arabinoside (AraC) [27]. It has been shown that this is a p53 dependent apoptosis [28]. We have measured the antiapoptotic effect of some amino acids, amino esters, amino phosphonic acids, amino nitriles and amino tetrazoles using this system.

Cultures of CGC were obtained from 6-8 day old Wistar rat pups. Cultures were grown on glass in 35 mm petri dishes for three days and then used for experiments. Aliquots (20 ul) of drug solutions (AraC, anti-apoptotic drugs, drug vehicles) were added to the medium of the culture. 24 Hours later the cultures were fixed with FAM, and stained with bis-benzamide. Normal and apoptotic nuclei were counted to a total of 90-120 cells per culture. The optimum concentration of AraC was found to be 300 uM.

The results are summarized in Tables 1 - 4.

**Table 1- Rescue by Amino Acids and Esters in the CGC Assay**

| | Compound | 10⁻⁶ M | 10⁻⁹ M | 10⁻¹²M | Rescue |
|---|---|---|---|---|---|
| Glycines (aminoacetic acids) | | | | | |
| | 2-Propyl | R | X | X | yes |
| | 1-Hexyl | X | R | X | yes |
| | R-2-Heptyl | X | X | X | no |
| | S-2-Heptyl | R | R | R | yes |

| B-Alanines (aminopropionic acids) | | | | | |
|---|---|---|---|---|---|
| | 2-Propyl | X | R | R | yes |
| | 2-Propyl-N-methyl | R | R | X | yes |
| | R-2-Pentyl | X | X | X | no |
| | 1-Hexyl | R | R | R | yes |
| | 1-Hexyl-N-methyl | R | R | X | yes |
| | R-2-Heptyl | | R | R | yes |
| | R-2-Heptyl-N-methyl | R | R | R | yes |
| | S-2-Heptyl | | | X | no |

| B-Alaninates (esters) | | | | | |
|---|---|---|---|---|---|
| | 2-Propyl | X | X | X | no |
| | 1-Hexyl | X | R | R | yes |
| | R-2-Heptyl-N-methyl | R | R | R | yes |

| | | | | | |
|---|---|---|---|---|---|
| R = rescue; X = no rescue | | | | | |

**Table 2 - Rescue by Amino Phosphonic Acids in the CGC Assay**

| Compound | 10⁻⁶M | 10⁻⁹ M | 10⁻¹² M | Rescue |
|---|---|---|---|---|
| 2-Propyl | R | R | R | yes |
| R-2-Pentyl | R | X | X | yes |
| 1-Hexyl | X | X | X | no |
| R-2-Heptyl | R* | X | X | yes* |
| S-2-Heptyl | X | R | R | yes |

| | | | | |
|---|---|---|---|---|
| * The presence of 0.5% S-enantiomer (which is potent at 10⁻¹² M) in the R-enantiomer is probably responsible for the apparent rescue by the R-enantiomer at 10⁻⁶ M. | | | | |

**Table 3 - Rescue by Amino Tetrazoles in the CGC Assay**

| Compound | 10⁻⁶M | 10⁻⁹M | 10⁻¹²M | Rescue |
|---|---|---|---|---|
| R-2-Heptyl | R | R | R | yes |
| S-2-Heptyl | X | X | X | no |

**Table 4 - Rescue by Amino Nitriles in the CGC Assay**

| Compound | | 10⁻⁶ M | 10⁻⁹ M | 10⁻¹²M | Rescue |
|---|---|---|---|---|---|
| Acetonitriles | | | | | |
| | 2-Propyl | R | R | R | yes |
| | R-2-Pentyl | X | R | X | yes |
| | S-2-Pentyl | X | X | X | no |
| | 1-Hexyl | X | R | R | yes |
| | R-2-heptyl | X | X | X | no |
| | S-2-Heptyl | X | R | R | yes |

| Propionitriles | | | | | |
|---|---|---|---|---|---|
| | 2-Propyl | X | X | X | no |
| | 2-Propyl-N-methyl | T | T | X | no |
| | R-2-Pentyl | X | X | X | no |
| | tert-Amyl | X | X | X | no |
| | tert-Amyl-N-methyl | X | X | X | no |
| | 3-Pentyl | R | R | X | yes |
| | 1-Hexyl | X | X | X | no |
| | 1-Hexyl-N-methyl | X | X | X | no |
| | R-2-Heptyl | X | R | R | yes |
| | R-2-Heptyl-N- | | | | |
| | methyl | X | R | X | yes |
| | S-2-heptyl | X | X | X | no |

| | | | | | |
|---|---|---|---|---|---|
| R = rescue; X = no rescue; T = toxic | | | | | |

### Detailed Synthetic Procedures

The following non-limiting examples of synthetic procedures are provided.

### Example 2:

### Methyl 3-(1-hexylamino)propionate hydrochloride [Methyl N-(1-hexyl)-β-alaninate]

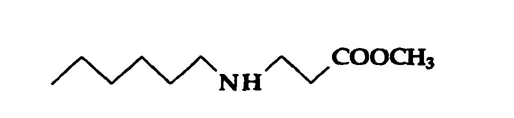

To ice-water-cooled 1-hexylamine (7.58 g, 75 mmol) was added dropwise methyl acrylate (4.3 g, 50 mmol). After completion of the addition the temperature was allowed to rise to room temperature and the reaction solution was stirred for 5 hours, then refluxed for 90 minutes. After stirring overnight at 20°C the product, methyl 3-(1-hexylamino)propionate, was distilled (b.p. = 85-88°C/2 mm; lit. b.p. 80-84°C/0.5 mm) as a clear, colorless liquid in a yield of 55%. The hydrochloride salt was prepared by the addition of methanolic HCl (15%) to an ethereal solution of the free base; m.p. = 204-205°C (lit. m.p. 190-192°C).
Mass spectrum: m/e: 187 (M+); 116 (M-C₅H₁₁); 84.

### Example 3:

### (R)-Methyl 3-(2-heptylamino)propionate hydrochloride [(R)-Methyl N-(2-heptyl)-β-alaninate]

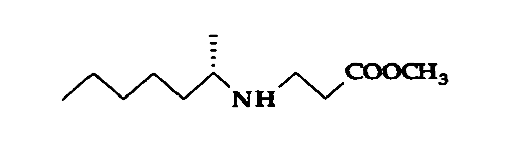

Prepared according to Example 2. The hydrochloride salt was recrystallized from methanol/ether; m.p. = 89.5-90.5°C.
Mass spectrum: m/e: 201 (M+); 186 (M-CH₃); 130 (M-C₅H₁₁)

### Example 4:

### Methyl 3-(2-propylamino)propionate hydrochloride [Methyl N-(2-propyl)-β-alaninate]

Prepared according to Example 2. The hydrochloride salt was crystallized from methanol/ether; m.p. = 108-110°C (lit. m.p. 107°C).
Mass spectrum: m/e: 145 (M+); 130 (M-CH₃); 98; 72; 56.

### Example 5:

### (R)-3-(2-Heptylamino)propionic acid hydrochloride [(R)-N-(2-heptyl)-β-alanine]

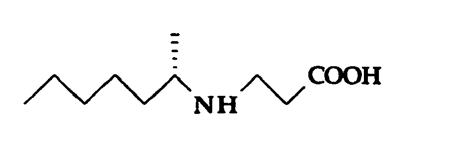

(R)-3-(2-Heptylamino)propionitrile (7.5 g, 44.6 mmol) (prepared according to Example 18) was refluxed with concentrated hydrochloric acid (50 mL) for 4 h. After filtration of insoluble ammonium chloride, the aqueous solution was rotary evaporated to dryness. The residue was stirred with dichloromethane (120 mL) for 2 hours and the insoluble ammonium chloride was filtered. The filtrate was concentrated, filtered again and then evaporated to give a colorless oil which crystallized on cooling. The yield of white hygroscopic product was quantitative; m.p. = 57-58°C.
Mass spectrum: m/e: 187 (M+); 172 (M-CH₃); 116 (M-C₅H₁₁); 72.
1H-NMR (D₂O, 300 MHz): 0.72 (t, 3H); 1.18 (d, 3H); 1.1-1.3 (m, 6H); 1.42/1.60 (2m, 1H each); 2.67 (t, 2H); 3.17 (m, 3H; αCH & CH₂COOH).

The title compound can also be prepared by hydrochloric acid hydrolysis of the methyl ester (prepared according to Example 3).

### Example 6:

### (S)-3-(2-Heptylamino)propionic acid hydrochloride [(S)-N-(2-heptyl)-β-alanine]

Prepared according to Example 5. The hydrochloride salt, m.p. = 56-58°C, is hygroscopic.
Mass spectrum: m/e: 187 (M+); 172 (M-CH₃); 128 (M-CH₂COOH); 116 (M-C₅H₁₁).
1H-NMR (D₂O, 300 MHz): 0.75 (t, 3H); 1.18 (d, 3H); 1.1-1.3 (m, 6H); 1.45/1.60 (2m, 1H each); 2.68 (t, 2H); 3.20 (m, 3H; αCH & CH₂COOH).

### Example 7:

### 3-(1-Hexylamino)propionic acid [N-(1-hexyl)-β-alanine]

Methyl 3-(1-hexylamino)propionate hydrochloride (see Example 2 for preparation) was hydrolyzed by refluxing in 2N HCl for 24 hours followed by evaporation to dryness; m.p. = 95-97°C (no lit. value).
Mass spectrum: m/e: 173 (M+);102 (M-C₅H₁₁); 84; 72.
1H-NMR (D₂O, 300 MHz): 0.73 (t, 3H); 1.17 (m, 6H); 1.52 (m, 2H); 2.68 (t, 2H); 2.93 (t, 2H); 3.18 (t, 2H).

### Example 8:

### 3-(2-Propylamino)propionic acid hydrochloride [N-(2-propyl)-β-alanine]

Prepared by hydrolysis of the ester (Example 7). The product is a white powder, m.p. = 154-155°C (no lit. value).
Mass spectrum: m/e: 131 (M+);116 (M-CH₃); 98; 56.
1H-NMR (D₂O, 300 MHz):1.20 (d, 6H); 270 (t, 2H); 3.19 (t, 2H); 3.32 (m, 1H).

### Example 9: (given for comparison)

### Ethyl 2-(2-propylamino)acetate hydrochloride [Ethyl N-(2-propyl)glycinate]

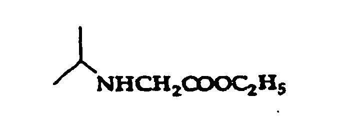

To a solution of 2-propylamine (4.4 g, 75 mmol) in ether (100 mL) was added ethyl bromoacetate (6.26g, 37.5 mmol). The solution was stirred at 20°C for 3 days. The precipitated 2-propylamine hydrobromide was filtered and the filtrate rotary evaporated to give 5.4 g a clear pale yellow liquid (crude yield = 99%). The hydrochloride salt was prepared and recrystallized from ethanol/ether; m.p. = 120-121°C (no lit. value).
Mass spectrum: m/e:145 (M+); 130 (M-CH₃); 72 (M-COOC₂H₅).

### Example 10: (given for comparison)

### 2-(2-Propylamino)acetic acid hydrochloride [N-(2-Propyl)glycine]

The sample was prepared from the nitrile (prepared according to Example 23) in 72% yield using a hydrolysis procedure similar to that described in Example 12. The hydrochloride salt was recrystallized from acetone; m.p. = 180-184°C (lit. m.p. 203-204.5°C; 182-183°C).
Mass spectrum: (CI). 118 (M+1); 102 (M-CH₃); 72 (M-CO₂H); (EI). 117 (M+); 102 (M-CH₃); 72 (M-COOH).
1H-NMR (D₂O, 300 MHz): 1.19 (d, 6H); 3.33 (m, 1H); 3.79 (s, 2H).

### Example 11: (given for comparison)

### 2-(1-Hexylamino)acetic acid hydrochloride [N-(1-Hexyl)glycine] 1HxActAc

The sample was prepared from the nitrile (procedure analogous to that described in Example 10) in 72% yield using a hydrolysis procedure similar to that described in Example 12. The hydrochloride salt was recrystallized from acetone; m.p.= 162-164°C (lit. m.p. 215-218°C).
Mass spectrum: m/e: 159 (M+); 114 (M-CO₂H); 88 (M-C₅H₁₁).
1H-NMR (D₂O, 300 MHz): 0.72 (t, 3H);1.20 (m, 6H);1.55 (m, 2H); 2.96 (t, 2H); 3.77 (s, 2H).

### Example 12: (given for comparison)

### (R)-2-(2-Heptylamino)acetic acid hydrochloride [(R)-N-(2-Heptyl)glycine]

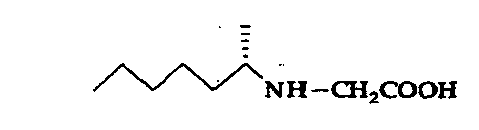

A solution of .(R)-2-(2-heptylamino)acetonitrile (1.20 g, 7.79 mmol) (prepared according to Example 23) in concentrated HCl (12 mL) and water (5 mL) was heated for 48 hours at 90°C. After cooling to room temperature, the reaction mixture was concentrated to dryness, filtered, and washed with ethanol to remove NH₄Cl. The resulting filtrate was concentrated to give a crude product (1.40 g). A solution of the crude product (0.60 g) in concentrated HCl (20 mL) and water (10 mL) was heated for 24 hours at 90°C. After cooling to room temperature, the reaction mixture was taken to dryness and triturated in ether to give the hydrochloride salt as a crystalline solid (0.60 g, overall 37% yield); m.p.= 162-164°C.
Mass spectrum: m/e: (CI). 174 (M+1); 158 (M-CH₃); 102 (M-C₅H₁₁).
1H-NMR (D₂O, 300 MHz): 0.74 (t, 3H);1.18 (d, 3H); 1.1-1.3 (m, 6H); 1.45 / 1.60 (2m, 1H each); 3.21 (m, 1H); 3.79 (s, 2H).

### Example 13: (given for comparison)

### (S)-2-(2-Heptylamino)acetic acid hydrochloride [(S)-N-(2-Heptyl)glycine]

The product was prepared as described in Example 12 in 72% overall yield by hydrolysis of the nitrile hydrochloride salt; m.p.= 161-163°C. Mass spectrum: m/e: (CI). 174 (M+1); 156 (M-OH); 128 (M-CO₂H).
1H-NMR (D₂O, 300 MHz): 0.73 (t, 3H); 1.18)d, m; 9H); 1.47, 1.60 (2m, 1H each); 3.21 (m, 1H); 3.78 (s, 2H).

### Example 14:

### Methyl 3-(2-propylmethylamino)propionate hydrochloride [Methyl N-(2-propyl)-N-methyl-β-alaninate]

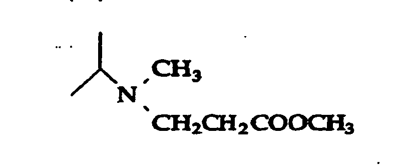

To a solution of methyl 3-(2-propylamino)propionate hydrochloride (Example 10)(0.907 g; 5 mmol) in 1N sodium dihydrogen phosphite (25 mL) was added 37% formaldehyde (2.1 mL, 23 mmol). The solution was stirred at 60°C for 10 min, then cooled in an ice-water bath and basified with 10% sodium hydroxide (10 mL). The resulting solution was saturated with sodium chloride (9 g) and immediately extracted with ether (3 x 15 mL). The combined filtrates were dried over anhydrous magnesium sulfate and evaporated to dryness to give a clear colorless liquid. The hydrochloride salt, prepared by the addition of methanolic HCl to an ether solution of the free base, precipitated as a viscous oil in 83 % yield.
Mass spectrum: m/e: 159 (M+); 144 (M-CH₃); 86 (M-CH₂COOCH₃).

### Example 15:

### (R)-Methyl 3-(2-heptylmethylamino)propionate hydrochloride [(R)-Methyl N-(2-heptyl)-N-methyl-β-alaninate]

The hydrochloride salt was obtained in 93% yield as a colorless viscous liquid.
Mass spectrum: m/e: 215 ((M+); 200 (M-CH₃); 144 (M-C₅H₁₁).

### Example 16:

### (R)-3-(2-Heptylmethylamino)propionic acid hydrochloride [N-(2-Heptyl)-N-methyl-β-alanine]

The hydrochloride salt precipitated as a colorless, viscous oil.
Mass spectrum: m/e: 201 (M+); 186 (M-CH₃); 130 (M-C₅H₁₁).
1H-NMR (D₂O, 300 MHz): 0.72 (t, 3H); 1.18 (m, 9H); 1.47, 1.59 (2m, 1H each); 2.65 (d, 3H); 2.74 (t, 2H); 3.11 (m, 1H); 3.34 (m, 2H).

### Example 17:

### (R)-3-(2-Heptylamino)propionitrile hydrochloride

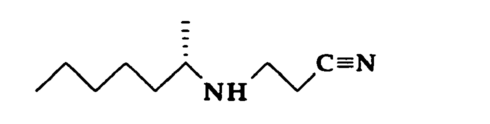

To ice-water-cooled (R)-2-heptylamine (99.4% R)(9.28 g, 80.7 mmol) was added dropwise acrylonitrile (2.85 g, 3.543 mL, 54 mmol). After completion of the addition the temperature was allowed to rise to room temperature and the reaction solution was stirred for 5 hours, then refluxed for 90 minutes. After stirring overnight at 20°C the product, (R)-3-(2-heptylamino)propionitrile, was distilled as a clear, colorless liquid, b.p. = 101-102°C/2 mm, with a yield of 85%. The hydrochloride salt was prepared by the addition of ethanolic HCl (15%) to an ethereal solution of the free base; m.p. = 134-135°C.
Mass spectrum: m/e: (CI) 169 (M+H)+; 153 (M-CH₃); 97 (M-C₅H₁₁).
1H-NMR (D₂O, 300 MHz): 0.73 (t, 3H); 1.20 (d, m; 9 H); 1.45, 1.61 (2m, 1H each); 2.85 (t, 2H); 3.3 (m, 3H).
Elemental Analysis: Calculated: %C = 58.66; %H = 10.34; %N = 13.68.
Found: %C = 58.73; %H= 10.53; %N= 13.47.

The starting material, (R)-2-heptylamine, was resolved from the racemate by repeated recrystallizations of its L-tartrate salt from methanol. Eight recrystallizations using a ratio of volume (of methanol) to weight (of tartrate salt) of 2.4 to 2.6 (increasing as optical purity increased) gave the R-enantiomer with an optical purity of 99.4% R. The optical purity was assessed by derivatization with freshly prepared chiral reagent (S)-N-trifluoroacetylprolyl chloride and then gas chromatography on a chiral column [23].

### Example 18:

### (S)-3-(2-Heptylamino)propionitrile hydrochloride

(S)-2-Heptylamine as its D-tartrate salt was prepared by recrystallization of the racemate (S-enriched, isolated and prepared from the combined filtrates of the R-enantiomer L-tartrate recrystallizations described in Example 17). The optical purity was 99.4% S. S-2HECN was prepared in 85% yield (b.p. 99-100°C/2 mm) as described for the R-enantiomer in Example 17. The hydrochloride salt was recrystallized from methanol/ether; m.p. = 133-134°C.
Mass spectrum: m/e: (CI) 169 (M+H)+; 153 (M-CH3); 128 (M-CH₂CN); 97 (M-C₅H₁₁).
1H-NMR (D₂O, 300 MHz): 0.72 (t, 3H); 1.20 (d, m; 9H); 1.45, 1.62 (2m, 1H each); 2.85 (t, 2H); 3.29 (m, 3H).
Elemental Analysis: Calculated: %C = 58.66; %H = 10.34; %N = 13.68.
Found: %C = 58.61; %H = 10.10; %N = 13.42.

### Example 19: (given for comparison)

### 3-(2-Propylamino)propionitrile hydrochloride

The free base, b.p. = 94°C/30 mm (lit. b.p. 86-87°C/17 mm), was prepared according to the procedure in Example 17 and then converted to the hydrochloride salt which was recrystallized from methanol/ether; m.p. = 145.5-146°C (no lit. value).
Mass spectrum: m/e: 140 (M+); 97 (M-CH₃); 72 (M-CH₂CN).
1H-NMR (D₂O, 300 MHz): 1.20 (d, 6H); 2.85 (t, 2H); 3.29 (t, 2H); 3.36 (m, 1H).

### Example 20: (given for comparison)

### 3-(1-Hexylamino)propionitrile hydrochloride

The hydrochloride salt was recrystallized from ethanol/ether;
m.p. = 188-189 °C (no lit. value).
Mass spectrum: m/e: 154 (M+); 114 (M-CH₂CN); 83 (M-C₅H₁₁).
1H-NMR (D₂O, 300 MHz): 0.72 (t, 3H); 1.18 (m, 6H); 1.53 (m, 2H); 2.87 (t, 2H); 2.96 (t, 2H); 3.29 (t, 2H).

### Example 21:

### 3-(3-Pentylamino)propionitrile hydrochloride

The free base, b.p. = 84-85°C/2 mm (no lit. value), was converted to the hydrochloride salt, m.p. = 118.5-119.5°C (no lit. value).
Mass spectrum: m/e: 140 (M+); 111 (M-C₂H₅); 100 (M-CH₂CN); 82; 70.
1H-NMR (D₂O, 300 MHz): 0.83 (t, 6H); 1.64 (m, 4H); 2.89 (t, 2H); 3.10 (m, 1H); 3,33 (t, 2H).

### Example 22: (given for comparison)

### 3-(t-Amylamino)propionitrile hydrochloride

The free base, b.p. = 62-63°C/2 mm (no lit. value), was converted to the hydrochloride salt, m.p. = 199-200°C (no lit. value).
Mass spectrum: m/e: 140 (M+, absent); 125 (M-CH₃); 111 (M-CH₂CH₃)
1H-NMR (D₂O, 300 MHz): 0.82 (t, 3H); 1.20 (s, 6H); 1.58 (q, 2H); 2.83 (t, 2H); 3.29 (t, 2H).

### Example 23: (given for comparison)

### (R)-2-(2-Heptylamino)acetonitrile hydrochloride

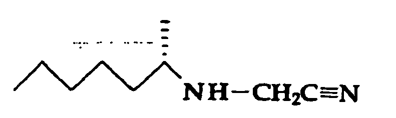

To a solution of (R)-2-heptylamine (1.90 g, 16.5 mmol) in ether (25 mL) were added anhydrous Na₂CO₃ (1.60 g, 14.9 mmol), and bromoacetonitrile (0.92 mL, 13.2 mmol). The reaction mixture was stirred at room temperature for 24 hours, and for another 14 hours at 80°C. After cooling to room temperature, the reaction mixture was filtered and the filtrate washed with HCl (3 N, 3x10 niL). The aqueous layer was basified with NaOH (6 N) and extracted with ether (3x25 mL). The resulting ethereal solution was dried over Na₂SO₄, and evaporated to dryness. The residue was fractionated by flash column chromatography (25% EtOAc/hexane, ether) to give a colorless oil (2.10 g, 82%). The hydrochloride salt was prepared by the addition of ethanolic HCl (15%) to an ethereal solution of the free base; m.p = 152-3°C
Mass spectrum: m/e: (CI).155 (M+1); _128 (M-CN); (EI) 1.39 (M-CH₃); 83 (M-C₅H₁₁).
1H-NMR (D₂O, 300 MHz): 0.75 (t, 3H); 1.20 (d, 3H); 1.15-1.35 (m, 6H); 1.47/1.62 (2m, 1H each); 3.35 (m, 1H); 4.18 (s, 2H).
Elemental Analysis: Calculated: %C = 56.68; %H = 10.04; %N = 14.69.
Found: %C = 56.81; %H = 10.20; %N = 14.46.

### Example 24: (given for comparison)

### (S)-2-(2-Heptylamino)acetonitrile hydrochloride

The sample was prepared in 96% yield using the above-described procedure (Example 23). The hydrochloride salt was prepared by the addition of ethanolic HCl (15%) to an ethereal solution of the free base and was recrystallized from ethanol/ether. The salt sublimes during melting; m.p. = 140°C.
Mass spectrum: m/e: 154 (M+);139 (M-CH₃).
1H-NMR (D₂O, 300 MHz): 0.73 (t, 3H); 1.18 (d, m; 9H); 1.43, 1.59 (2m, 1H each); 3.31 (m, 1H); 4.15 (s, 2H).
Elemental Analysis: Calculated: %C = 56.68; %H = 10.04; %N = 14.69.
Found: %C = 56.51; %H = 9.71; %N = 14.79.

### Example 25: (given for comparison)

### 2-(2-Propylamino)acetonitrile hydrochloride

The hydrochloride salt was recrystallized from methanol/ether;
m.p. = 166-167°C (lit. m.p. 154-156°).
Mass spectrum: m/e: 98 (M+); 83 (M-CH₃); 56.
1H-NMR (D₂O, 300 MHz): 1.19 (d, 6H); 3.45 (m,1H); 4.17 (s, 2H).

### Example 26: (given for comparison)

### 2-(1-Hexylamino)acetorutrile hydrochloride

The free base of the sample was prepared in 62% yield using the above-described procedure (Example 23). The hydrochloride salt was prepared by the addition of ethanolic HCl (15%) to an ethereal solution of the free base, and was recrystallized from ethanol/ether; m.p.= 114-115°C (lit. m.p. 84-86°C).
Mass spectrum: m/e: (CI).141 (M+1)+; 126 (M-CN).
1H-NMR (D₂O, 300 MHz): 0.74 (t, 3H); 1.21 (m, 6H); 1.58 (m, 2H); 3.08 (t, 2H); 4.17 (s,2H).

### Example 27:

### (R)-3-(2-Heptylmethylamino)propionitrile hydrochloride

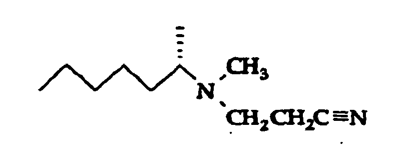

(R)-3-(2-Heptylamino)propionitrile (Example 17) (0.85 g, 5.0 mmol) was dissolved in-1N sodium dihydrogen phosphite (NaH₂PO₃)(25 mL) and 37% formaldehyde (2.1 mL, 23 mmol) was added. Sufficient dioxan was added to give a clear solution (10 mL). The solution was stirred at 60°C for 15 min during which time it became cloudy. To the cooled solution was then added 20% sodium hydroxide (20 mL) and sodium chloride (9 g). The basic solution was extracted with ether (3 x 15 mL). The combined extracts were dried over anhydrous magnesium sulfate and then evaporated to give a clear, colorless liquid in quantitative yield. The hydrochloride salt was prepared by adding methanolic HCl to an ether solution of the free base; m.p. = 98-98.5°C. Overall yield was 85%.
Mass spectrum: m/e: 182 (M+); 167 (M-CH₃); 142 (M-CH₂CN); 111 (M-C₅H₁₁).
1H-NMR (D₂O, 300 MHz): 0.77 (t, 3H); 1.20 (d, 3H); 1.15-1.35 (m, 6H); 1.50/1.62 (2m, 1H each); 273 (s, 3H); 2.95 (t, 2H); 3.40 (m, 3H; aCH & CH₂CN).
Elemental Analysis: Calculated: %C = 60.39; %H = 10.60; %N = 12.80.
Found: %C = 59.55; %H = 10.29; %N = 13.67.

### Example 28: (given for comparison)

### 3-(2-Propylmethylamino)propionitrile hydrochloride

The hydrochloride salt was obtained in 90% yield; m.p. = 121-121.5°C (no lit. value)(see Example 27).
Mass spectrum: m/e: 126 (M+); 111 (M-CH₃); 86 (M-CH₂CN).
1H-NMR (D₂O, 300 MHz): 1.23 (d, 6H); 2.73 (s, 3H); 2.97 (t, 2H); 3.52 (br s, 2H); . 3.60 (m, 1H).

### Example 29: (given for comparison)

### 3-(t-Amylmethylamino)propionitrile hydrochloride

The hydrochloride salt was obtained in 87% yield; m.p. = 137-138°C (no lit. value)(see Example 27).
Mass spectrum: m/e: 154 (M+); 139 (M-CH₃);125 (M-C₂H₅); 72.
1H-NMR (D₂O, 300 MHz): 0.87 (t,3H); 1.25 (6H); 1.67 (q, 2H); 2.10 (s, 2H); 2.73 (s, 3H); 2.94 (broad t, 2H).
Elemental Analysis: Calculated: %C = 56.68; %H = 10.04; %N = 14.69.
Found: %C = 56.62; %H =10.12; %N = 14.62.

### Example 30: (given for comparison)

### 3-(1-Hexylmethylamino)propionitrile hydrochloride

The hygroscopic hydrochloride salt was obtained in 75% yield;
m.p. = 77-78.5°C (no lit. value)(see Example 27).
Mass spectrum: m/e: 168 (M+); 128 (M-CH₂CN); 97 (M-C₅H₁₁).
1H-NMR (D₂O, 300 MHz): 0.72 (t, 3H); 1.20 (m, 6H); 1.60 (m, 2H); 2.79 (s, 3H); 2.95 (t, 2H); 3.09 (t, 2H); 3.42 (t, 2H).

### Example 31: (given for comparison)

### Diethyl 2-(2-propylamino)ethanephosphonate hydrochloride

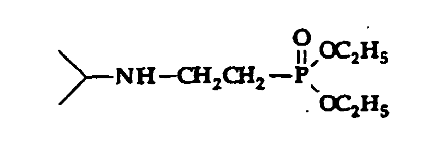

To ice-cooled 2-propylamine (0.53 mL, 6.2 mmol) was added dropwise diethyl vinylphosphonate (0.4 mL, 3.1 mmol) under N₂. The reaction mixture was stirred for 2 hours at 0°C, for 14 hours at room temperature, and for 10 hours at 100°C. After cooling to room temperature, the reaction mixture was concentrated to give a colorless oil (0.89 g). To a solution of the crude product (0.89 g) in ether (40 mL) was added ethanolic HCl (15%), and then stirred for 2 hours at room temperature. The resulting crystallized hydrochloride salt was filtered, washed with ether to give a white solid (0.76 g, overall 95%); m.p. = 96-97°C (no lit. value).
Mass spectrum: m/e: 208 (M-CH₃)+; 180 (M-C₃H₇)+.

### Example 32:

### 2-(2-Propylamino)ethanephosphortic acid hydrochloride

The hydrochloride salt of the product (starting from the diester, Example 31) was prepared in 32% overall yield using the procedure described in Example 37; m.p.= 164-166°C (no lit. value).
Mass spectrum: m/e: (CI). 168 (M+1)+.
1H-NMR (D₂O, 300 MHz): 1.16 (d, 6H); 1.84 (m, 2H); 3.08 (q, 2H); 3.27 (m,1H).

### Example 33: (given for comparison)

### Diethyl 2-(1-hexylamino)ethanephosphonate hydrochloride

The crude product was prepared in 100% yield using the procedure as described in Example 31. The hydrochloride salt of the product was prepared by the addition of ethanolic HCl (15%) to an ethereal solution of the product; the salt is a viscous oil.
Mass spectrum: m/e: 265 (M)+, 194 (M-C₅H₁₁)+.

### Example 34: (given for comparison)

### 2-(1-Hexylamino)ethanephosphonic acid hydrochloride

The hydrochloride salt was prepared by hydrolysis of the diethyl ester using the method described in Example 37; m.p. = 145-150°C (no lit. value).
Mass spectrum: m/e: too involatile
1H-NMR (D₂O, 300 MHz): 0.70 (t, 3H); 1.15 (m, 6H); 1.50 (m, 2H); 1.90 (m, 2H); 2.88 (t, 2H); 3.08 (q, 2H).
Elemental Analysis: Calculated: %C = 39.11; %H =:8.62; %N = 5.70.
Found: %C = 39.34; %H = 8.53; %N = 5.89.

### Example 35:

### (R)-Diethyl 2-(2-heptylamino)ethanephosphonate hydrochloride

The free base was prepared in 93% yield using the procedure as described in Example 31. The hydrochloride salt was prepared by the addition of ethanolic HCl (15%) to an ethereal solution of the product; the salt is a viscous oil.
Mass spectrum: m/e: (CI). 280 (M+1)+; 264.(M-CH₃)+; 208 (M-C₅H₁₁)+.

### Example 36:

### (S)-Diethyl 2-(2-heptylamino)ethanephosphonate hydrochloride

The free base was prepared in 91% yield using the procedure as described in Example 31. The hydrochloride salt was prepared by the addition of ethanolic HCl (15%) to an ethereal solution of the product; the salt is a viscous oil.
Mass spectrum: m/e: (CI). 280 (M+1)+; 208 (M-C₅H₁₁)+.

### Example 37:

### (R)-2-(2-Heptylamino)ethanephosphonic acid hydrochloride

A solution of (R)-diethyl 2-(2-heptylamino)ethanephosphonate hydrochloride (0.19 g, 0.68 mmol) in concentrated HCl (7 mL) was heated at 90°C for 48 hours. The reaction mixture was then evaporated to dryness and the residue triturated with acetone. The white solid was filtered and air-dried giving an 86% overall yield; m.p. = 106-112°C (no lit. value).
Mass spectrum: m/e: too involatile
1H-NMR (D₂O, 300 MHz): 0.73 (t, 3H); 1.25-1.10 (m, 12H); 1.43 (m, 1H); 1.58 (m, 1H);
1.89-1.78 (m, 2H); 3.22-3.05 (m, 3H).
Elemental Analysis: Calculated: %C = 41.62; %H = 8.93; %N = 5.39.
Found: %C = 41.68; %H = 9.10; %N = 5.23.

### Example 38:

### (S)-2-(2-Heptylamino)ethanephosphonic acid hydrochloride

The hydrochloride salt was prepared in 71% overall yield using the above described procedure; m.p. = 106-113°C (no lit. value).
Mass spectrum: m/e: too involatile
1H-NMR (D₂O, 300 MHz): 0.73 (t, 3H); 1.25-1.10 (m, 12H); 1.44 (m, 1H); 1.59 (m, 1H); 1.89-1.78 (m, 2H); 3.22-3.05 (m, 3H).
Elemental Analysis: Calculated: %C = 41.62; %H = 8.93; %N = 5.39.
Found: %C = 41.43; %H = 9.09; %N = 5.33.

### Example 39:

### (R)-2-(2-Heptylamino)ethane-5-tetrazole hydrochloride

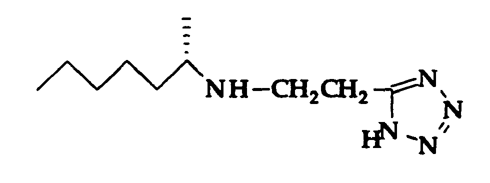

The compound as its hydrochloride salt was prepared in 28% overall yield using the procedure described below in Example 40; the salt is a viscous oil.
Mass spectrum: m/e: (CI). 140 (M-C₅H₁₁)+; 128 (M-C₂H₃N₄)+.
1H-NMR (D₂O, 300 MHz): 0.71 (t, 3H); 1.18 (m, 9H); 1.44, 1.60 (2m, 1H each); 3.30 (t+m; 3H); 3.45 (m, 2H).

### Example 40:

### (S)-2-(2-Heptylamino)ethane-5-tetrazole hydrochloride

To an ice-cooled suspension of NaN₃ (2.60 g, 40 mmol) in dry THF (20 mL) was added AlCl₃ (1.36 g, 10 mmol) in portions under N₂, and stirred for 30 minutes. To the resulting suspension was added a solution of (S)-3-(2-heptylamino)propionitrile (1.68 g, 10 mmol) in THF (10 mL), and stirred for 2 hours at 0°C, then gently refluxed for 24 hours. After cooling to room temperature, the reaction mixture was quenched by the careful addition of HCl (3 N, 15 mL), water (5 mL), and the two layers were separated. The lower aqueous layer was extracted with ethyl acetate (3x15 mL). The upper (organic) layer and the organic extracts were combined and dried over Na₂SO₄, then taken to dryness to give crude product (oil, 1.36 g). The oil was diluted with ether (30 mL) and ethanol (5 mL), stirred for two hours, and filtered to give the hydrochloride salt (0.21 g, 9% overall yield);
m.p. = 112-113°C (no lit. value).
Mass spectrum: m/e: (CI). 140 (M-C₅H₁₁)+; 128 (M-C₂H₃N₄)+.
1H-NMR (D20, 300 MHz): 0.72 (t, 3H); 1.20 (d, 3H); 1.15-1.35 (m, 6H); 1.45/1.62 (2m, 1H each); 3.30 (t+m; 3H); 3.43 (m, 2H).
Elemental analysis: Calculated: %C = 48.47; %H = 8.95; %N = 28.27.
Found: %C = 48.24; %H = 8.85; %N = 28.40.

### FULL CITATIONS FOR REFERENCES REFERRED TO IN THE SPECIFICATION

1. Ansari, K.S., et al., Rescue of axotomized immature rat facial motoneurons by R(-)-deprenyl: stereospecificity and independence from monoamine oxidase inhibition. Journal of Neuroscience, 1993. 13: p. 4042-4053.
2. Davis, B.A., et al. Neurorescue by the optically active enantiomers of some aliphatic N-methylpropargylamines. Abstract, American Society for Neurochemistry. 1995. Santa Monica, CA.
3. Oh, C., et al., (-)-Deprenyl alters the survival of adult murine facial motoneurons after axotomy: Increases in vulnerable C57BL strain but decreases in motor neuron degeneration mutants. J. Neurosci. Res., 1994. 38: p. 64-74.
4. Paterson, I.A., B.A. Davis, and A.A. Boulton, Aliphatic propargylamines prevent hippocampal neuronal death induced by hypoxia-ischemia. J. Neurochem., 1997. 69 (Supp): p. S137.
5. Paterson, LA., et al., (-)-Deprenyl reduces delayed neuronal death of hippocampal pyramidal cells. Neurosci. Biobehav. Rev., 1997. 21: p.181-186.
6. Paterson, I.A., et al., R-Deprenyl and R-2-heptyl-N-methylpropargylamine prevent apoptosis in cerebellar granule neurons induced by cytosine arabinoside but not low extracellular potassium. J. Neurochem., 1998. 98: p. 515-523.
7. Paterson, LA., et al., The anti-apoptotic effects of 2HMP is due to a desmethyl metabolite. Society for Neuroscience Abstracts, 1997. 23 (part 2): p. 2254 (#880.6).
8. Paterson, LA., et al., Aliphatic propargylamines as cellular rescue agents. United States Patent, Filed July 14,1997.
9. Tatton, W.G. and C.E. Greenwood, Rescue of dying neurons: a new action for deprenyl in MPTP Parkinsonism. J. Neurosci. Res., 1991. 30: p. 666-672.
10. Tatton, W.G., et al., (-)-Deprenyl reduces PC12 cell apoptosis by inducing new protein synthesis. J. Neurochem., 1994. 63: p. 1572-1575.
11. Salo, P.T. and W.G. Tatton, Deprenyl reduces the death of motoneurons caused by axotomy. J. Neurosci. Res., 1992. 31: p. 394-400.
12. Wu, R.-M., D.L. Murphy, and C.C. Chiueh, Neuronal protective and rescue effects of deprenyl against MPP+ dopaminergic toxicity. J. Neural Transm. [Gen Sect], 1995. 100: p. 53-61.
13. Yoles, E. and M. Schwartz, N-Propargyl-1 (R)-aminoindan (TVP-1012), a putative neuroprotective agent, enhance in vitro neuronal survival after glutamate toxicity. Abstract, American Society for Neuroscience. 1995. San Diego, CA.
14. Zhang, X., et al., Immunohistochemical evidence of neuroprotection by R-(-)-deprenyl 1 and N-(2-hexyl)-N-methylpropargylamine on DSP-4-induced degeneration of rat brain noradrenergic axons and terminals. Journal of Neuroscience Research, 1996. 43: p. 482-489.
15. Yu, P.H., Davis, B.A., Boulton, A.A., Aliphatic propargylamines as specific MAO-B inhibitors and as neuroprotective agents. United States Patent No. 5,508,311 (1992).
16. Durden, D.A., et al., Aliphatic propargylamines as cellular rescue agents. United States Patent No. 5,840,979 (1997).
17. Grace, J.M., M.T. Kinter, and T.L. Macdonald, Atypical metabolism of deprenyl and its enantiomer, (S)-(+)-N,alpha-dimethyl-N-propynylphenylethylamine, by cytochrome P450 2D6. Chem. Res. Toxicol., 1994. 7: p. 286-290.
18. Komives, E.A. and P.R. Ortiz de Montellano, Mechanism of oxidation of π bonds by cytochrome P-450. J. Biol. Chem., 1987. 262: p. 9793-9802.
19. Roberts, E.S., et al., Mechanism-based inactivation of cytochrome 450 2B1 by 9-ethynylphenanthrene. Arch. Biochem. Biophys., 1995. 323: p. 295-302.
20. Valoti, M., et al., Interactions between substituted tryptamine analogues, MAO inhibitors and cytochrome P-450. J. Neural Transm. [Suppl], 1994. 41: p. 291-293.
21. Tarbell, D.S., et al., The synthesis of some 7-chloro-4-(3-alkylaminopropylamino)quinolines. J. Am. Chem. Soc., 1946. 68: p. 1217-1219.
22. Mazur, R.H., Absolute configuration of 1-methylalkylamines. J. Organic Chemistry, 1970. 35: p. 2050-2051.
23. Durden, D.A., B.A. Davis, and A.A. Boulton, Enantioselective gas chromatographic assay of 2-alkylamines using N-(trifluoroacetyl)propyl derivatives and a chiral capillary column. Journal of Chromatography B, 1997. 689: p. 165-173.
24. Robinson, J.B. and J. Thomas, The preparation of N-t-butyl-4-piperidone. J. Chem. Soc., 1965: p. 2270-2271.
25. Arnold, C. and D.N. Thatcher, Preparation and reactions of 5-vinyltetrazole. J. Org. Chem., 1969. 34: p. 1141-1142.
26. Loibner, H., A. Pruckner, and A. Stutz, Reductive methylation of amines. Tetrahedron Lett., 1984. 25: p. 2535-2536.
27. Dessi, F., et al., Cytosine arabinoside induces apoptosis in cerebellar neurons in culture. J. Neurochem., 1995. 64: p. 1980-1987.
28. Enokido, Y., et al., P53 involves cytosine arabinoside induced apoptosis in cultured cerebellar granule neurons. Neurosci. Lett., 1996. 203: p. 1-4.

## Claims

1. A compound of the Formula I 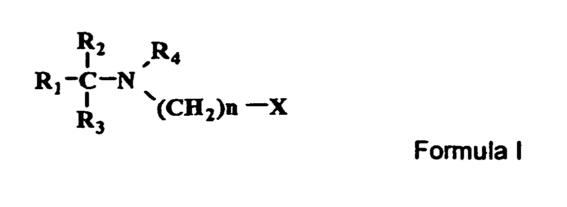 wherein:
R₁ is (CH₂)ₘCH₃ where m is 0 or an integer in the range from 1 to 16,
R₂ = CH₃,
R₃ = H,
R₄ = H or CH₃,
n is 2,
X is carboxyl (COOH) or carbalkoxy (COOR₅), cyano (CN), phosphonic acid (PO₃H₂), phosphonate ester (PO₃[R₅]₂) or 5-tetrazole,
R₅ is lower alkyl having from 1 to 5 carbon atoms, and
R₁, R₂ and R₃ differ from one another so that the carbon atom to which they are attached is chiral and the compound of Formula I is a substantially pure enantiomer in the R configuration when X is COOH, COOR₅, CN or tetrazole, or a substantially pure enantiomer in the S configuration when X is PO₃H₂ or PO₃[R₅]₂,
or a pharmaceutically acceptable salt thereof.

2. A compound of the Formula I according to claim 1 wherein:
X is carboxyl (COOH), carbalkoxy (COOR₅), cyano (CN) or 5-tetrazole, and
R₅ = lower alkyl having from 1 to 5 carbon atoms,
as a substantially pure enantiomer in the R-configuration, or a pharmaceutically acceptable salt thereof.

3. A compound of the Formula I according to claim 1 wherein:
X is phosphonic acid (PO₃H₂) or phosphonate ester (PO₃[R₅]₂), and
R₅ = lower alkyl having from 1 to 5 carbon atoms,
as a substantially pure enantiomer in the S-configuration, or a pharmaceutically acceptable salt thereof.

4. A compound according to claim 2 wherein said compound of Formula I is selected from the group consisting of:
(R)-3-(2-Heptylamino)propionic acid;
(R)-3-(2-Heptylmethylamino)propionic acid;
Methyl (R)-3-(2-heptylamino)propionate;
Methyl (R)-3-(2-heptylmethylamino)propionate;
(R)-3-(2-Heptylamino)propionitrile;
(R)-3-(2-Heptylmethylamino)propionitrile; and
(R)-2-(2-Heptylamino)ethane-5-tetrazole.

5. A compound according to claim 3 wherein said compound of Formula I is selected from the group consisting of:
(S)-2-(2-Heptylamino)ethanephosphonic acid; and
(S)-2-(2-Heptylmethylamino)ethanephosphonic acid.

6. A compound selected from the group consisting of:
3-(2-Propylmethylamino)propionic acid;
Methyl 3-(1-hexylmethylamino)propionate;
3-(3-Pentylamino)propionitrile;
3-(3-Pentylmethylamino)propionitrile;
2-(2-Propylamino)ethanephosphonic acid; and
2-(2-Propylmethylamino)ethanephosphonic acid.

7. A compound according to any one of claims 1 to 6 in the form of a hydrochloride salt.

8. A compound according to any one of claims 1 to 3 wherein m is an integer from 1 to 12.

9. A compound according to any one of claims 1 to 3 wherein m is an integer from 1 to 9.

10. A composition for the treatment or prevention of a disease in which cell death occurs by apoptosis, which composition comprises an effective amount of a compound having the Formula I as claimed in any one of claims 1-9.

11. A compound of the Formula I as claimed in any one of claims 1-9 for the use in medicine.

12. A use of a compound of the Formula I as claimed in any one of claims 1-9 for the manufacture of a medicament for the treatment or prevention of a disease in which cell death occurs by apoptosis.

13. A use of a compound selected from:
3-(2-Propylamino)propionic acid;
3-(1-Hexylamino)propionic acid;
3-(2-Propylmethylamino)propionic acid;
3-(1-Hexylmethylamino)propionic acid;
2-(2-Propylamino)ethanephosphonic acid; and
2-(2-Propylmethylamino)ethanephosphonic acid,
for the manufacture of a medicament for the treatment or prevention of a disease in which cell death occurs by apoptosis.

14. A use according to claim 12 or 13 wherein the disease is selected from the group consisting of stroke, head trauma, Bell's palsy, spinal cord injury, Alzheimer's disease, Parkinson's disease, Pick's disease, amyotrophic lateral sclerosis, Huntington's disease, multiple sclerosis, cardiac myopathies, nephropathy, retinopathy, diabetic complications, glaucoma and idiopathic neuropathies.

15. A use according to any one of claims 12 to 14, for the manufacture of a medicament for the treatment of a human.

16. A commercial package for the treatment or prevention of a disease in which cell death occurs by apoptosis, said package comprising a pharmaceutical composition according to claim 10, together with instructions for use in the treatment or prevention of diseases in which cell death occurs by apoptosis.

## Patentansprüche

1. Verbindung der Formel I 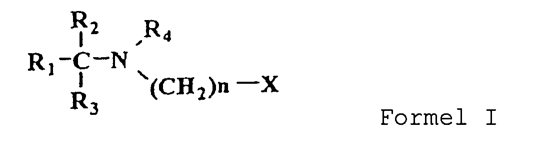 worin:
R₁ für (CH₂)ₘCH₃ steht, worin m für 0 oder eine ganze Zahl im Bereich von 1 bis 16 steht,
R₂ = CH₃,
R₃ = H,
R₄ = H oder CH₃,
n für 2 steht,
X für Carboxyl (COOH) oder Carbalkoxy (COOR₅), Cyano (CN), Phosphonsäure (PO₃H₂), Phosphonatester (PO₃[R₅]₂) oder 5-Tetrazol steht,
R₅ für Niederalkyl mit 1 bis 5 Kohlenstoffatomen steht und
R₁, R₂ und R₃ sich voneinander unterscheiden, so dass das Kohlenstoffatom, an das sie gebunden sind, chiral ist und die Verbindung der Formel I ein im Wesentlichen reines Enantiomer in der R-Konfiguration ist, wenn X für COOH, COOR₅, CN oder Tetrazol steht, oder ein im Wesentlichen reines Enantiomer in der S-Konfiguration ist, wenn X für PO₃H₂ oder PO₃[R₅]₂ steht,
oder ein pharmazeutisch annehmbares Salz davon.

2. Verbindung der Formel (I) gemäß Anspruch 1, worin:
X für Carboxyl (COOH), Carbalkoxy (COOR₅), Cyano (CN) oder 5-Tetrazol steht, und
R₅ = Niederalkyl mit 1 bis 5 Kohlenstoffatomen,
als ein im Wesentlichen reines Enantiomer in der R-Konfiguration, oder ein pharmazeutisch annehmbares Salz davon.

3. Verbindung der Formel I gemäß Anspruch 1, wobei:
X für Phosphonsäure (PO₃H₂) oder Phosphonatester (PO₃[R₅]₂) steht und
R₅ = Niederalkyl mit 1 bis 5 Kohlenstoffatomen,
als ein im Wesentlichen reines Enantiomer in der S-Konfiguration, oder ein pharmazeutisch annehmbares Salz davon.

4. Verbindung gemäß Anspruch 2, wobei die Verbindung der Formel I ausgewählt ist aus der Gruppe, bestehend aus:
(R)-3-(2-Heptylamino)propionsäure;
(R)-3-(2-Heptylmethylamino)propionsäure;
Methyl-(R)-3-(2-heptylamino)propionat;
Methyl-(R)-3-(2-heptylmethylamino)propionat;
(R)-3-(2-Heptylamino)propionitril;
(R)-3-(2-Heptylmethylamino)propionitril; und
(R)-2-(2-Heptylamino)ethan-5-tetrazol.

5. Verbindung gemäß Anspruch 3, wobei die Verbindung der Formel I ausgewählt ist aus der Gruppe, bestehend aus:
(S)-2-(2-Heptylamino)ethanphosphonsäure; und
(S)-2-(2-Heptylmethylamino)ethanphosphonsäure.

6. Verbindung, ausgewählt aus der Gruppe, bestehend aus:
3-(2-Propylmethylamino)propionsäure;
Methyl-3-(1-hexylmethylamino)propionat;
3-(3-Pentylamino)propionitril;
3-(3-Pentylmethylamino)propionitril;
2-(2-Propylamino)ethanphosphonsäure; und
2-(2-Propylmethylamino)ethanphosphonsäure.

7. Verbindung gemäß einem der Ansprüche 1 bis 6 in der Form eines Hydrochloridsalzes.

8. Verbindung gemäß einem der Ansprüche 1 bis 3, wobei m für eine ganze Zahl von 1 bis 12 steht.

9. Verbindung gemäß einem der Ansprüche 1 bis 3, wobei m für eine ganze Zahl von 1 bis 9 steht.

10. Zusammensetzung zur Behandlung oder Prävention einer Erkrankung, bei der Zelltod durch Apoptose geschieht, wobei die Zusammensetzung eine wirksame Menge einer Verbindung der Formel I gemäß einem der Ansprüche 1-9 umfasst.

11. Verbindung der Formel I gemäß einem der Ansprüche 1-9 zur Verwendung in der Medizin.

12. Verwendung einer Verbindung der Formel I gemäß einem der Ansprüche 1-9 zur Herstellung eines Arzneimittels zur Behandlung oder Prävention einer Erkrankung, bei der Zelltod durch Apoptose geschieht.

13. Verwendung einer Verbindung, ausgewählt aus:
3-(2-Propylamino)propionsäure;
3-(1-Hexylamino)propionsäure;
3-(2-Propylmethylamino)propionsäure;
3-(1-Hexylmethylamino)propionsäure;
2-(2-Propylamino)ethanphosphonsäure; und
2-(2-Propylmethylamino)ethanphosphonsäre
zur Herstellung eines Arzneimittels zur Behandlung oder Prävention einer Erkrankung, bei der Zelltod durch Apoptose geschieht.

14. Verwendung gemäß Anspruch 12 oder 13, wobei die Erkrankung ausgewählt ist aus der Gruppe, bestehend aus Schlaganfall, Schädeltrauma, Bell-Lähmung, Rückenmarksverletzung, Alzheimerscher Krankheit, Parkinsonscher Krankheit, Pickscher Krankheit, amyotropher Lateralsklerose, Huntingtonscher Krankheit, Multipler Sklerose, Kardiomyopathien, Nephropathie, Retinopathie, diabetischen Komplikationen, Glaukom und idiopathischen Neuropathien.

15. Verwendung gemäß einem der Ansprüche 12 bis 14 zur Herstellung eines Arzneimittels zur Behandlung eines Menschen.

16. Handelspackung zur Behandlung oder Prävention einer Erkrankung, bei der Zelltod durch Apoptose geschieht, wobei die Packung eine pharmazeutische Zusammensetzung gemäß Anspruch 10 zusammen mit Anweisungen zur Verwendung in der Behandlung oder Prävention von Erkrankungen, bei denen Zelltod durch Apoptose geschieht, umfasst.

## Revendications

1. Composé de Formule I: 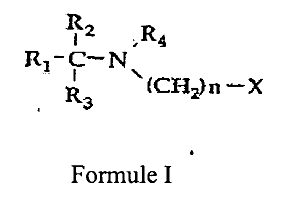 dans laquelle:
- R₁ est (CH₂)ₘCH₃ où m est 0 ou un entier compris dans la gamme de 1 à 16,
- R₂ est CH₃,
- R₃ est H,
- R₄ est H ou CH₃,
- n est 2,
- X est un groupe carboxy (COOH) ou carbalcoxy (COOR₅), cyano (CN), acide phosphonique (PO₃H₂), ester phosphonate (PO₃[R₅]₂) ou 5-tétrazole,
- R₅ est un groupe alkyle inférieur ayant 1 à 5 atomes de carbone, et
- R₁, R₂ et R₃ diffèrent les uns des autres de telle sorte que l'atome de carbone auquel ils sont attachés est chiral et que le composé de Formule I est un énantiomère substantiellement pur en configuration R lorsque X est COOH, COOR₅, CN ou tétrazole, ou un énantiomère substantiellement pur en configuration S lorsque X est PO₃H₂ ou PO₃[R₅]₂,
ou sel de celui-ci acceptable du point de vue pharmaceutique.

2. Composé de Formule I selon la revendication 1, dans lequel:
- X est un groupe carboxy (COOH) ou carbalcoxy (COOR₅), cyano (CN) ou 5-tétrazole, et
- R₅ est un groupe alkyle inférieur ayant 1 à 5 atomes de carbone,
sous la forme d'un énantiomère substantiellement pur en configuration R, ou d'un sel de celui-ci acceptable du point de vue pharmaceutique.

3. Composé de Formule I selon la revendication 1, dans lequel:
- X est un groupe acide phosphonique (PO₃H₂) ou ester phosphonate (PO₃[R₅]₂), et
- R₅ est un groupe alkyle inférieur ayant 1 à 5 atomes de carbone,
sous la forme d'un énantiomère substantiellement pur en configuration S, ou d'un sel de celui-ci acceptable du point de vue pharmaceutique.

4. Composé selon la revendication 2, dans lequel ledit composé de Formule 1 est choisi dans le groupe consistant en:
acide (R)-3-(2-heptylamino)-propionique,
acide (R)-3-(2-heptylméthylamino)-propionique,
(R)-3-(2-heptylamino)-propionate de méthyle,
(R)-3-(2-heptylméthylamino)-propionate de méthyle,
(R)-3-(2-heptylamino)-propionitrile,
(R)-3-(2-heptylméthylamino)-propionitrile et
(R)-2-(2-heptylamino)-éthane-5-tétrazole.

5. Composé selon la revendication 3, dans lequel ledit composé de Formule I est choisi dans le groupe consistant en:
acide (S)-2-(2-heptylamino)-éthanephosphonique et
acide (S)-2-(2-heptylméthylamino)-éthanephosphonique.

6. Composé choisi dans le groupe consistant en:
acide 3-(2-propylméthylamino)-propionique,
3-(1-hexylméthylamino)-propionate de méthyle,
3-(3-pentylamino)-propionitrile,
3-(3-pentylméthylamino)-propionitrile,
acide 2-(2-propylamino)-éthanephosphonique et
acide 2-(2-propylméthylamino)-éthanephosphonique.

7. Composé selon l'une quelconque des revendications 1 à 6 sous la forme d'un chlorhydrate.

8. Composé selon l'une quelconque des revendications 1 à 3, dans lequel m est un entier de 1 à 12.

9. Composé selon l'une quelconque des revendications 1 à 3, dans lequel m est un entier de 1 à 9.

10. Composition pour le traitement ou la prévention d'une maladie dans laquelle il apparaît une mort cellulaire par apoptose, laquelle composition comprend une quantité efficace d'un composé ayant la Formule I selon l'une quelconque des revendications 1 à 9.

11. Composé selon l'une quelconque des revendications 1 à 9 utile en médecine.

12. Utilisation d'un composé de Formule 1 selon l'une quelconque des revendications 1 à 9 pour la fabrication d'un médicament destiné au traitement ou à la prévention d'une maladie dans laquelle une mort cellulaire apparaît par apoptose.

13. Utilisation d'un composé choisi parmi les suivants:
acide 3-(2-propylamino)-propionique,
acide 3-(1-hexylamino)-propionique,
acide 3-(2-propylméthylamino)-propionique,
acide 3-(1-hexylméthylamino)-propionique,
acide 2-(2-propylamino)-éthanephosphonique et
acide 2-(2-propylméthylamino)-éthanephosphonique
pour la fabrication d'un médicament destiné au traitement ou à la prévention d'une maladie dans laquelle une mort cellulaire apparaît par apoptose.

14. Utilisation selon la revendication 12 ou 13, dans laquelle la maladie est choisie dans le groupe consistant en accident vasculaire cérébrale, trauma de la tête, paralysie de Bell, lésion de la moelle épinière, maladie d'Alzheimer, maladie de Parkinson, maladie de Pick, sclérose latérale amyotrophique, maladie de Huntington, sclérose en plaques, myopathies cardiaques, néphropathie, rétinopathie, complications diabétiques, glaucome et neuropathies idiopathiques.

15. Utilisation selon l'une quelconque des revendications 12 à 14, pour la fabrication d'un médicament pour le traitement d'un humain.

16. Conditionnement commercial pour le traitement ou la prévention d'une maladie dans laquelle une mort cellulose apparaît par apoptose, ledit conditionnement comprenant une composition pharmaceutique selon la revendication 10, comportant des instructions utiles pour le traitement ou la prévention des maladies dans lesquelles une mort cellulaire apparaît par apoptose.
